Europäisches Patentamt

European Patent Office

Office européen des brevets

(19)

(11) Veröffentlichungsnummer: **0 131 060**
**B1**

(12) **EUROPÄISCHE PATENTSCHRIFT**

(45) Veröffentlichungstag der Patentschrift: **25.04.90**

(51) Int. Cl.⁵: **G 01 R 29/12, A 61 N 1/14**

(21) Anmeldenummer: **83106720.2**

(22) Anmeldetag: **08.07.83**

(54) Gerät zur partiellen Feststellung von Spannungen oder Ladungen am menschlichen Körper sowie zum Ableiten derartiger Spannungen oder Ladungen vom menschlichen Körper.

(43) Veröffentlichungstag der Anmeldung:
16.01.85 Patentblatt 85/03

(45) Bekanntmachung des Hinweises auf die
Patenterteilung: 25.04.90 Patentblatt 90/17

(84) Benannte Vertragsstaaten:
AT BE CH DE FR GB IT LI LU NL SE

(56) Entgegenhaltungen:
DE-A-2 823 542
DE-A-3 118 062
US-A-3 214 706

FUNKSCHAU, Band 45, Nr. 14, 1973, München C.
ROCKROHR "Elektronisches Elektroskop", Seite
531

(73) Patentinhaber: **Esper, Herbert, Dipl.-Ing. (FH)**
**Bürgermeister-Heinrich-Strasse 23**
**D-8403 Bad Abbach (DE)**

(72) Erfinder: **Esper, Herbert, Dipl.-Ing. (FH)**
**Bürgermeister-Heinrich-Strasse 23**
**D-8403 Bad Abbach (DE)**

EP 0 131 060 B1

**Beschreibung**

Die Erfindung betrifft ein Gerät zur partiellen Feststellung von Spannungen oder Ladungen an einem menschlichen oder tierischen Körper mit einem als Verstärker oder als elektrisch steuerbarer Schalter wirkenden Element mit einem Arbeitsstromkreis und einem Steuerstromkreis in Form einer Transistoranordnung, wobei der Steuerstromkreis einen Steuernschluß sowie einen für de Steuerstromkreis und Arbeitsstromkreis gemeinsamen Anschluß aufweist, une eine als Sonde dienende Elektrode, die mit dem Steueranschluß verbunden ist, und eine durch den Strom im Arbeitsstromkreis angesteuerte Anzeigevorrichtung vorgesehen ist.

Es ist heute allgemein anerkannt, daß an der Hautoberfläche von Personen, und zwar in den verschiedensten Körperregionen Spannungen bzw. Konzentrationen von Ladungsträgern auftreten können, wobei diese partiellen Spannungen bzw. ladungsträgerkonzentrationen zur Vermeidung von gesundheitlichen Störungen nicht nur abgeleitet werden müssen, sondern aus diesen partiellen Spannungen bzw. Ladungsträgerkonzentrationen lassen sich — in Abhängigkeit von den Körperpartien, an denen diese Spannungen bzw. Ladungsträgerkonzentrationen auftreten — auch Rücklüsse auf kranke oder anfällige Körperteile bzw. -organe ziehen.

Der Erfindung liegt die Aufgabe zugrunde, ein Gerät aufzuzeigen, mit welchem es möglich ist, partielle Spannungen oder Ladungen an einen menschlichen Körper abzuleiten, wobei das Gerät die Möglichkeit eröffnet, diesen Entladungsvorgang und damit auch das vollständige Ableiten von Spannungen oder Ladungen anzuzeigen sowie diejenigen Körperregionen zu bestimmen, an denen partielle Spannungen oder Ladungsträgerkonzentrationen vorhanden sind, um so ggf. Rückschlüsse auf kranke oder anfällige Körperteile bzw. -organze ziehen zu können.

Es ist bereits aus dem Artikel Funkschau, Heft 14, 1973, Seite 531 ein elektronisches Elektroskop bekannt, welches ebenfalls zur Anzeige von elektrischen Ladungen dient. Es ist ebenfalls mit einem als Verstärker oder als elektrisch steuerbarer Schalter wirkendem Element mit einem Arbeitsstromkreis und einem Steuerstromkreis in Form einer Transistorschaltung aufgebaut. Dabei weist der Steuerstromkreis einen Steueranschluß sowie einen für den Steuerstromkreis und Arbeitsstromkreis gemeinsamen Anscluß auf, sowie einen als Sonde dienenden Fühler, der mit dem Steueranschluß verbunden ist.

Es ist ebenfalls im Arbeitsstromkreis eine Anzeigevorrichtung vorgesehen. Bei dieser bekannten Anordnung ist jedoch der Steueranscluß oder Fühler völlig freischwebend, so daß keine Mittel vorhanden sind, die bei fehlender äußerer Spannung an der Sonde den Steuerschluß auf einem vorgegebenen Spannungsniveau halten.

Zur Lösung dieser Aufgabe ist ein Gerät der eingangs geschilderten Art erfindungsgemäß gekennzeichnet durch Mittel, die bei fehlender äußerer Spannung an der Sonde den Steueranschluß auf einem vorgegebenen Spannungsniveau halten.

Bei einer bevorzugten Ausführungsform des erfindungsgemäßen Gerätes sind diese letzgenannten Mittel so ausgebildet, daß sie bei fehlender äußerer Spannung an der Sonde im Steuerenschluß des als Verstärker oder als elektronisch steuerbarer Schalter wirkenden Elements auf einem Spannungsniveau halten, welches keinen Strom im Arbeitsstromkreis oder aber in diesem Arbeitsstromkreis nur einen Strom in solcher Größer zu Folge hat, daß die Anzeigevorrichtung zu keiner Anzeige führt. Die das Spannungsniveau des Steueranschlusses bei fehlender äußerer Spannung an der Sonde bzw. die Vorspannung des Steuerschlusses festlegenden Mittel sind dann beispielsweise im einfachsten Fall von einem hochohmigen Widerstand oder von einer Zener-Diode gebildet, der bzw. die im Steuerstromkreis bevorzugt parallel zu dem Steueranschluß und dem für den Steuerstromkreis und dem Arbeitsstromkreis gemeinsamen Anschluß liegt.

Bei einer besonders einfachen Ausführungsform des erfindungsgemäßen Gerätes ist daß als Verstärker oder elektronisch steuerbarer Schalter wirkende Element wenigstens ein Transistor, dessen Basis dann den Steueranschluß und dessen Emitter den für den Steuerstromkreis und Arbeitsstromkreis gemeinsamen Anschluß bilden.

Die Anzeigevorrichtung befindet sich dann bevorzugt im Kollektorstromkreis dieses Transistors oder aber im Kollektor-oder Emitter-Stromkreis eines weiteren, nachgeschalteten Transistors oder aber im Ausgangsstromkreis eines dem ersten Transistor nachgeschalteten Verstärkerelementes.

Die Anzeigevorrichtung ist bevorzugt von einer Leuchdiode gebildet.

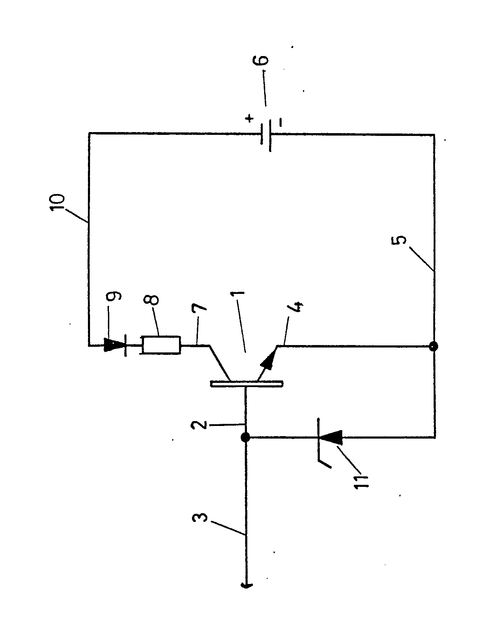

Die Erfindung wird im folgenden im Zusammenhang mit der Figur, die ein Ausführungsbeispiel zeigt, näher erläutert.

In der Figur ist 1 ein Transistor des npn-Typs. Die Basis 2 dieses Transistors ist direkt an das eine Ende einer als Sonde dienenden Elektrode 3 angeschlossen, deren anderes, freies Ende zum Feststellen und Ableiten von Spannungen oder Ladungsträgern mit der Hautoberfläche eines menschlichen Körpers in Berührung gebracht werden kann. Die Sonde 3 besteht selbstverständlich aus elektrisch leitendem Material, wobei zumindest die Oberfläche dieser Sonde von einem korrosionsbeständigem Material (z.B. Silver) gebildet ist. Der Emitter 4 des Transistors 1 ist über eine Leitung 5 mit dem Minus-Pol einer Batterie 6 verbunden. Der Kollektor 7 steht über die Reihenschaltung eines Schutzwiederstandes 8 und einer als Anzeigevorrichtung diendenden Leuchtdiode 9 über die Leitung 10 mit dem Plus-Pol der Batterie 6 in Verbindung.

Parallel zur Basis-Emitter-Strecke des Transistors 1 liegt eine Zener-Diode, die einen sehr hochohmigen Basis-Ableit-Widerstand bildet.

Werden nun durch Abtasten der Hautoberfläche eines menschlichen Körpers an einer bestimmten Stelle gegenüber dem Emitter positive Spannungen festgestellt, so wird der normalerweise geschlossene Transistor 1 geöffnet, wodurch im Kollektor-Emitter-Stromkreis ein Strom durch die Leuchdiode 9 fließt und diese solange zum Aufleuchten bringt, bis die Spannungen an der betreffenden Körperstelle vollständig abgebaut sind, und zwar hauptsächlich über die Basis-Emitter-Strecke des Transistors 1. Nach dem Abbau bzw. Albeiten dieser Spannungen wird der Transistor 1 weider in seine sperrende Stellung überfeführt und die Leuchtdiode erlischt.

Die Erfindung wurde voranstehend an einem Ausführungsbeispiel beschrieben. Es versteht sich, daß Änderungen sowie Abwandlungen möglich sind, ohne daß dadurch der der Erfindung zugrundeliegende Erfindungsgedanke verlassen wird. So ist es beispielsweise möglich, anstelle der Zenerdiode 11 eien hochohmigen Widerstand zu verwenden, wenngleich eine Zenerdiode hinsichtlich einer besseren Reproduzierbarkeit vorteilhafter ist.

Weiterhin ist es selbstverständlich auch möglich, anstelle eines npn-Transistors bei entsprechender Umkehrung der Polung der Batterie 6, der Leuchtdiode 9 sowie der Zenerdiode 11 einen Transistor 1 vom pnp-Typ zu verwenden, wobei in diesem Fall negative Spannungen an der Elektrode 1 ein Ansprechen der Leuchtdiode 9 bewirken.

Schließlich ist es auch möglich, anstelle des in der Figur dargestellten Transistors 1 einen Feldeffekt-Transistor einzusetzen, der sich durch einen besonders hohen Eingangswiderstand auszeichnet.

## Patentansprüche

1. Gerät zur partiellen Feststellung von Spannungen oder Ladungen an einem menschlichen oder tierischen Körper mit einem als Verstärker oder als elektrisch steuerbarer Schalter wirkenden Element (1) mit einem Arbeitsstromkreis und einem Steuerstromkreis in Form einer Transistoranordnung, wobei der Steuerstromkreis einen Steueranschluß sowie einen für den Steuerstromkreis und Arbeitsstromkreis gemeinsamen Anschluß (4) aufweist, une eine als Sonde dienende Elektrode (3), die mit dem Steueranschluß (2) verbunden ist, und eine durch den Strom im Arbeitsstromkreis angesteuerte Anzeigevorrichtung (9) vorgesehen ist, gekennzeichnet durch Mittel (11), die bei fehlender äußerer Spannung an der Sonde (3) den Steueranschluß (2) auf einem vorgegebenen Spannungsniveau halten.

2. Gerät nach Anspruch 1, dadurch gekennzeichnet, das das als Verstärker oder als elektronisch steuerbarer Schalter dienende Element ein Transistor (1) ist.

3. Gerät nach Anspruch 1 oder 2, dadurch gekennzeichnet, daß die Mittel, die bei fehlender äußerer Spannung an der Sonde (3) den Steueranschluß (2) auf einem vorgegebenen Spannungsniveau halten, von wenigstens einer Diode, vorzugsweise von einer Zenerdiode (11) gebildet sind.

4. Gerät nach einem der Ansprüche 1 bis 3, dadurch gekennzeichnet, daß die Mittel, die bei fehlender äußerer Spannung an der Sonde (3) den Steueranschluß (2) auf einem vorgegebenen Spannungsniveau halten, von wenigstens einem Widerstand gebildet sind.

5. Gerät nach einem der Ansprüche 1 bis 4, dadurch gekennzeichnet, daß die Mittel, die bei fehlender äußerer Spannung an der Sonde (3) den Steueranschluß (2) auf einem vorgegebenen Spannungsniveau halten, parallel zur Steuerstrecke bzw. parallel zum Steueranschluß (2) und dem für den Steuerstromkreis und den Arbeitsstromkreis gemeinsamen Anschluß (4) liegen.

6. Gerät nach einem der Ansprüche 1 bis 5, dadurch gekennzeichnet, daß die Anzeigevorrichtung eine Leuchtdiode, vorzugsweise eine mit einem Schutzwiderstand (8) in Reihe liegende Leuchtdiode (9) ist.

7. Gerät nach einem der Ansprüche 2 bis 6, dadurch gekennzeichnet, daß der Steueranschluß von der Basis eines Transistors (1) gebildet ist.

8. Gerät nach einem der Ansprüche 2 bis 7, dadurch gekennzeichnet, daß der für den Steuerstromkreis und den Arbeitsstromkreis gemeinsame Anschluß von dem Emitter (4) eines Transistors (1) gebildet ist.

## Revendications

1. Appareil de détermination partielle de tensions ou charges sur un corps humain ou animal, comprenant, d'une part, un élément (1) qui sert d'amplificateur ou d'interrupteur à commande électrique et auquel sont associés un circuit de travail et un circuit de commande, sous la forme d'un montage du type transistor, le circuit de commande comportant une borne de commande et une borne (4) commune à ce circuit de commande et au circuit de travail, et, d'autre part, une électrode (3) qui sert de sonde et qui est branchée sur la borne de commande (2), tandis qu'il est prévu un dispositif de visualisation (9) qui est commandé par le courant circulant dans le circuit de travail, caractérisé par des moyens (11) qui maintiennent la borne de commande (2) à un niveau préfixé de tension lorsqu'il n'existe pas de tension extérieur sur la sonde (3).

2. Appareil suivant la revendication 1, caractérisé en ce que l'élément servant d'amplificateur ou d'interrupteur à commande électronique est un transistor (1).

3. Appareil suivant la revendication 1 ou 2, caractérisé en ce que les moyens qui maintiennent la borne de commande (2) à un niveau préfixé de tension lorsqu'il n'existe pas de tension extérieur sur la sonde (3) sont constitués par au moins une diode, de préférence une diode Zener (11).

4. Appareil suivant l'une des revendications 1 à 3, caractérisé en ce que les moyens qui maintiennent la borne de command (2) à un niveau préfixé de tension lorsqu'il n'existe pas de tension extérieure sur la sonde (3) sont constitués par au moins un résistance.

5. Appareil suivant l'une des revendications 1 à 4, caractérisé en ce que les moyens qui maintiennant la borne de commande (2) à un niveau préfixé de tension lorsqu'il n'existe pas de tension extérieure sur la sonde (3) sont montés en parallèle au tronçon de commande ou en parallèle entre la borne de commande (2) et la borne (4) commune au circuit de commande et au circuit de travail.

6. Appareil suivant l'une des revendications 1 à 5, caractérisé en ce que le dispositif de visualisation est un diode lumineuse, de préférence une diode lumineuse (9) montée en série avec une résistance protectrice (8).

7. Appareil suivant l'une des revendications 2 à 6, caractérisé en ce que la borne de commande est constituée par la base d'un transistor (1).

8. Appareil suivant l'une des revendications 2 à 7, caractérisé en ce que la borne commune au circuit de commande et au circuit de travail est constituée par l'émetteur (4) d'un transistor (1).

**Claims**

1. A device for the partial determination of voltages or charges at a human or animal body, having an element (1) acting as an amplifier or as an electrically controllable switch, having an operating circuit and a control circuit in the form of a transistor arrangement, the control circuit having a control connection and a connection (4) common to the control circuit and the operating circuit, and an electrode (3) serving as a probe, which electrode (3) is connected to the control connection (2), and a display device (9) driven by the current in the operating circuit, characterised by means (11) which maintain the control connection (2) at a predetermined voltage level in the event of a lack of external voltage at the probe (3).

2. A device according to Claim 1, characterised in that the element serving as an amplifier or electronically controllable switch is a transistor (1).

3. A device according to Claim 1 or 2, characterised in that the means, which maintain the control connection (2) at a predetermined voltage level in the event of a lack of external voltage at the probe (3) are constructed of at least one diode, preferably a Zener diode (11).

4. A device according to any one of Claims 1 to 3, characterised in that the means which maintained the control connection (2) at a predetermined voltage level in the event of a lack of external voltage at the probe (3) are constructed of at least one resistor.

5. A device according to any one of Claims 1 to 4, characterised in that the means which maintained the control connection (2) at a predetermined voltage level in the event of a lack of external voltage at the probe (3) are arranged parallel to the control path or parallel to the control connection (2) and the connection (4) common to the control circuit and the operating circuit.

6. A device according to any one of Claims 1 to 5, characterised in that the display device is a light emitting diode, preferably a light emitting diode (9) arranged in series with a protective resistor (8).

7. A device according to any one of Claims 2 to 6, characterised in that the control connection is constructed of a base of a transistor (1).

8. A device according to any one of Claims 2 to 7, characterised in that the connection common to the control circuit and the operating circuit is constructed of the emitter (4) of a transistor (1).